# EUROPEAN PATENT SPECIFICATION

(11) **EP 2 022 325 B1**
(45) Date of publication and mention of the grant of the patent: **27.10.2010**
(21) Application number: 07114106.3
(22) Date of filing: 09.08.2007
(51) Int. Cl.: A01K 11/00, A61B 5/07

(54) **Method of tagging an animal**
Verfahren zur Kennzeichnung eines Tieres
Procédé de marquage d'un animal

(43) Date of publication of application: 11.02.2009
(73) Proprietor: The European Union, represented by the European Commission, 1049 Brussels (BE)
(72) Inventor: Lungu, Sorin, 21017 Ispra (VA) (IT); Fiore, Gianluca, 21100 Varese (IT)
(74) Representative: Office Freylinger

(56) References cited:
- EP-A- 0 808 574
- WO-A-90/05448
- US-A1- 2005 234 475

## Description

### Technical field

The present invention generally relates to tagging an animal, more specifically to such tagging by means of transponder, which is remotely electronically interrogatable.

### Background Art

Livestock identification systems (using ear tags, tattoos, ruminal boluses, injectable transponders and/or the like) play a key role for veterinary monitoring as well as livestock and meat product traceability. The technologies currently available for electronic identification of livestock still fail to satisfactorily fulfil certain basic requirements. In particular, there should be no migration of transponder within the body of the tagged animal, transponder losses should be avoided, the possibility of fast recovery of the transponders in the slaughterhouses should be ascertained. Ideally the transponders should also be usable in the second part of the meat production chain, e.g. for the identification of meat products or the animal carcasses.
In the last years, a certain number of experiments have been carried out, for example, under the Fifth Framework Programme of the European Commission, as documented in "EID+DNA Tracing, EC, The Fifth Framework Programme, QLK1-2001-02229, 2003" (available from quiro.uab.es/tracing/Reports/-1stAnnualReport/1stAnnual%20Report.pdf).

As implantation sites (in pigs) have been considered: the auricle base, the abdominal cavity, the shinbone region, the ciliary arch and the armpit. In general, the above reports concluded that a) the loss rate varied from 0% to 50%, b) recovery was always incompatible with the average speed of pig meat processing chain, and finally c) the subcutaneous technique is not recommended for the electronic identification of pigs.

Further implantation sites that have been investigated (for pigs) are the nasal zone and the rostral zone (region rostralis dorsalis). For reference, see "Subcutaneous nasal transponder implant, an alternative method for electronic identification of pigs - preliminary results", S. Lungu, G. Fiore, C. Korn; 56th Annual Meeting of the EAAP, Uppsala, Sweden, 2005; "Subcutaneous transponder implant in pigs - results of a comparative field trail concerning tagging procedures and reading performance", Lungu S., Fiore G.; USAMV International Symposium Animal Science Timisoara 2006 and "Subcutaneous implant of transponder in pig's rostral zone (Region Rostralis Dorsalis) - histological reaction at the slaughter age", Lungu S., Fiore G.; USAMV International Symposium Animal Science Timisoara 2006.

According to an alternative method developed by Dr. James D. McKean (USA) for electronic identification of pigs, the transponder is placed subcutaneous under the dewclaw of the right hind leg.

### Technical problem

It is an object of the present invention to provide a method for tagging an animal with an electronically readable transponder, which is compatible with the requirements of modern livestock management. This object is achieved by a method as claimed in claim 1.

### General Description of the Invention

According to the invention, a method for tagging an animal comprises the injection of an electronically readable transponder into the calcaneal area of the animal.

The present technique represents a positive answer to the requirements of absence of migration within the body of the animal (due to the local anatomical characteristics at the calcaneum), low transponder loss risk, easy and efficient trace-back of the animal and the product(s) to the farm of origin (each animal being individually tagged and thus identifyable). In addition, at the proposed implantation site the transponder is easily readable with state-of-the-art reading equipment. Furthermore, the area where the transponder is injected does not per se affect the proper functioning of the transponder; the implantation site is easily accessible, making the reading of the implanted transponder fast and accurate.

In addition, the method allows identifying the animal and its carcass throughout the slaughtering process. Since the implantation site is in a region of the animal that does not enter the food chain, nothing opposes to leaving the electronic transponder in place during the entire meat production chain (from the implantation, which may be short after the birth of the animal, to the final product purchased by the consumer). The present method thus provides a single solution for the identification of the living animal, in the slaughtering chain and of the meat product. With specific regard to ham production (when the transponder is implanted in a pig), the present method gives the possibility to leave the transponder in position during the entire slaughtering process so that the fresh ham and possibly also the processed ham will be identifiable and fully back-traceable to the herd of origin.

Preferably, by the injection, the transponder is positioned on the external, latero-posterior side of the calcaneum, in a vertical position, substantially parallel with the vertical axis of the calcaneum. As used herein, the term "vertical" refers to the direction of the body axis of the animal that is vertical when the animal is standing or moving on its feet. According to the most preferred embodiment of the invention, the transponder is implanted with a needle, the needle being introduced about at the middle of the metatarsus V bone, rear side, and then moved along the metatarsus V bone in the direction of the calcaneum until the tip of the needle is at the base of the calcaneum. When the needle is in place, the transponder is deposited through the needle.

Most preferably, the transponder is injected in such a way as to have a substantially vertical orientation when said animal is standing (or moving).

Those skilled will appreciate that the present method is applicable, in particular, to pigs, cattle and sheep. Preferably the animal subjected to the tagging is a piglet, a calf or a lamb. The transponder preferably has a length of about 11 to 23 mm for a piglet and a length of about 32 mm for a calf or a lamb.

### Brief Description of the Drawings

A preferred embodiment of the invention will now be described, by way of example, with reference to the accompanying drawings in which:

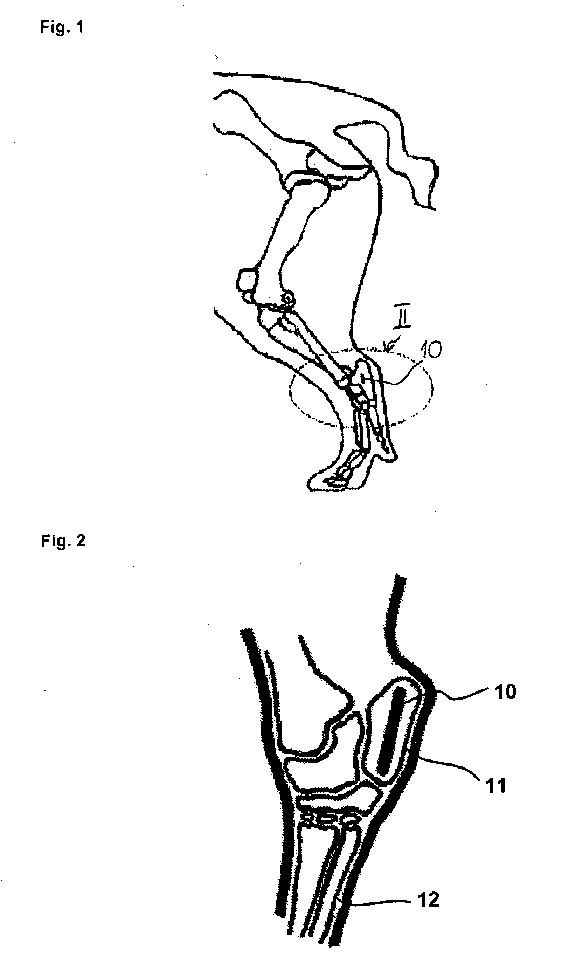

Fig. 1 is a schematic view of the implantation site of a transponder in a pig's leg;
Fig. 2 is a view of detail II of Fig. 1 (the hock region of the pig).

### Description of a Preferred Embodiment

For electronically tagging a piglet, the implant, i.e. the electronically readable transponder 10, is placed on the external - lateral side of calcaneum 11, in a parallel position with the vertical axis of the calcaneum. More precisely, the injection is performed along the metatarsus V bone, indicated at 12. The needle is moved subcutaneous, along the vertical axis of the metatarsus V bone 12, into the latero-plantar area of the calcaneum 11, preferably so that the transponder 10 reaches the top of the calcaneum 11, through dilacerations of the subcutaneous tissue. The transponder 10 thus comes to lie in a substantially parallel position with respect to the vertical axis of the calcaneum 11, on the latero-plantar side thereof.

Two people, one restraining the piglet and one implanting the transponder, can easily tag the animal. Both actions could be carried out by a single person, when the piglet's age is between one day and two weeks. For piglets less than 24 hours old, the method allows implanting a transponder with a length between 11 and 23 mm.

## Claims

1. Method of tagging an animal, comprising the insertion of an electronically readable transponder into the animal, **characterised in that** the transponder is injected into the calcaneal area of the animal.

2. Method according to claim 1, wherein, by said injection, the transponder is positioned substantially in parallel with the vertical axis of the calcaneum.

3. Method according to claim 1 or 2, wherein the transponder is positioned on the latero-plantar side of the calcaneum.

4. Method according to any one of claims 1 to 3, wherein said transponder is injected in such a way as to have a substantially vertical orientation when said animal is standing.

5. Method according to any of the previous claims, wherein the transponder is injected with a needle, wherein said injection comprises Introducing said needle about at the middle of the metatarsus V bone, rear side, then moving said needle along said metatarsus V bone in the direction of the calcaneum until the point of said needle is at the base of the calcaneum and deposit said transponder through said needle.

6. Method according to any of the previous claims, wherein said animal is a piglet, a calf or a lamb.

7. Method according to claim 6, wherein the transponder has a length of about 11 to 23 mm for a piglet and a length of about 32 mm for a calf or a lamb.

## Patentansprüche

1. Verfahren zur Kennzeichnung eines Tieres, umfassend die Einbringung eines elektronisch lesbaren Transponders in das Tier, **dadurch gekennzeichnet, dass** der Transponder in den Kalkaneusbereich des Tieres injiziert wird.

2. Verfahren nach Anspruch 1, wobei der Transponder durch die Injektion im Wesentlichen parallel zur Vertikalachse des Kalkaneus positioniert wird

3. Verfahren nach Anspruch 1 oder 2, wobei der Transponder an der lateroplantaren Seite des Kalkaneus positioniert wird

4. Verfahren nach irgendeinem der Ansprüche 1 bis 3, wobei der Transponder derart injiziert wird, dass er eine im Wesentlichen vertikale Ausrichtung aufweist, wenn das Tier steht

5. Verfahren nach irgendeinem der vorangehenden Ansprüche, wobei der Transponder mit einer Nadel injiziert wird, wobei die Injektion Folgendes umfasst
Einführen der Nadel ungefähr an der Mitte des Metatarsalknochens V, Rückseite; dann Bewegen der Nadel entlang des Metatarsalknochens V in Richtung des Kalkaneus, bis sich die Spitze der Nadel an der Basis des Kalkaneus befindet; und Absetzen des Transponders durch die Nadel hindurch

6. Verfahren nach irgendeinem der vorangehenden Ansprüche, wobei das Tier ein Ferkel, ein Kalb oder ein Lamm ist

7. Verfahren nach Anspruch 6, wobei der Transponder eine Länge von ungefähr 11 bis 23 mm für ein Ferkel und eine Länge von ungefähr 32 mm für ein Kalb oder ein Lamm aufweist

## Revendications

1. Procédé de marquage d'un animal, comprenant l'insertion d'un transpondeur à lecture électronique dans l'animal, **caractérisé en ce que** le transpondeur est injecté dans la zone du calcanéum de l'animal.

2. Procédé selon la revendication 1, dans lequel, par ladite injection, le transpondeur est positionné substantiellement en parallèle avec l'axe vertical du calcanéum

3. Procédé selon la revendication 1 ou 2, dans lequel le transpondeur est positionné sur le côté latéro-plantaire du calcanéum

4. Procédé selon l'une quelconque des revendications 1 à 3, dans lequel ledit transpondeur est injecté de façon à avoir une orientation substantiellement verticale lorsque ledit animal est debout.

5. Procédé selon l'une quelconque des revendications précédentes, dans lequel le transpondeur est injecté avec une aiguille, dans lequel ladite injection comprend
l'introduction de ladite aiguille environ au milieu de l'os du métatarse V, côté arrière, puis le déplacement de ladite aiguille le long dudit os du métatarse V dans la direction du calcanéum jusqu'à ce que la pointe de ladite aiguille soit à la base du calcanéum et le dépôt dudit transpondeur à travers ladite aiguille

6. Procédé selon l'une quelconque des revendications précédentes, dans lequel ledit animal est un porcelet, un veau ou un agneau

7. Procédé selon la revendication 6, dans lequel le transpondeur a une longueur d'environ 11 à 23 mm pour un porcelet et une longueur d'environ 32 mm pour un veau ou un agneau.
